# EUROPÄISCHE PATENTSCHRIFT

(11) **EP 0 960 911 B1**
(45) Veröffentlichungstag und Bekanntmachung des Hinweises auf die Patenterteilung: **24.07.2002**
(21) Anmeldenummer: 99109909.4
(22) Anmeldetag: 20.05.1999
(51) Int. Cl.: C09B 67/00, C09D 5/36, C08K 9/02, C08K 7/00

(54) **Pigmentmischung**
Pigment mix
Mélange pigmentaire

(30) Priorität: 28.05.1998 DE 19823864
(43) Veröffentlichungstag der Anmeldung: 01.12.1999
(73) Patentinhaber: MERCK PATENT GmbH, 64293 Darmstadt (DE)
(72) Erfinder: Vogt, Reiner Dr., 64289 Kranichstein (DE); Maurer, Uta, 64293 Darmstadt (DE); Schül, Norbert, 64646 Heppenheim (DE); Schoen, Sabine Dr., 64287 Darmstadt (DE); Osterried, Karl Dr., 64807 Dieburg (DE)

(56) Entgegenhaltungen:
- WO-A-95/30546
- WO-A-98/03583
- DE-A- 4 240 511

## Beschreibung

Die vorliegende Erfindung betrifft Pigmentmischungen bestehend aus mindestens zwei Komponenten, wobei Komponente A mit ein oder mehreren Metalloxiden und/oder Metallen beschichtete SiO₂-Flakes und Komponente B plättchenförmige, nadelförmige oder sphärische Farbmittel oder Füllstoffe sind, sowie deren Verwendung in Lacken, Farben, Druckfarben, Kunststoffen, Pulverlacken und kosmetischen Formulierungen.

Deckvermögen und Glanz sind bei plättchenförmigen Pigmenten oftmals nur schwer gleichzeitig in befriedigendem Ausmaß zu realisieren. So zeichnen sich etwa mit einer oder mehreren dünnen Metalloxidschichten belegte Glimmerplättchen oder SiO₂-Flakes durch Interferenzfarben und einen hohen Glanz, gleichzeitig aber auch wegen des durchsichtigen Substrats durch eine hohe Transparenz und damit ein vergleichsweises geringes Deckvermögen, aus.

Aus der DE-A-42 40 511 ist eine Pigmentmischung bekannt, die sich aus einem Interferenzpigment und einem plättchenförmigen Farbpigment zusammensetzt. Das Interferenzpigment sind mit Metalloxiden beschichtete Glimmer- oder SiO₂-Flakes und das Farbpigment können farbige SiO₂-Flakes sein. Dieses Pigmentgemisch wird in Lacke, Druckfarben oder Kunststoffe eingearbeitet.

Aufgabe der vorliegenden Erfindung ist es eine Pigmentmischung bereitzustellen, die sich durch ein vergleichsweise hohes Deckvermögen auszeichnet und sich gut in das jeweilige Anwendungssystem einarbeiten läßt und bei der gleichzeitig eine Trennung Pigment/Farbmittel im System weitgehend ausgeschlossen ist.

Überraschenderweise wurde nun eine Pigmentmischung gefunden, die keine der oben angegebenen Nachteile aufweist. Das erfindungsgemäße Pigmentgemisch besteht aus mindestens zwei Komponenten, wobei Komponente A mit ein oder mehreren Metalloxiden und/oder Metallen beschichtete SiO₂-Flakes und Komponente B plättchenförmige, nadelförmige oder sphärische Farbmittel oder Füllstoffe sind. Durch die Zumischung des Farbmittels zu den beschichteten SiO₂-Flakes kann den Anwendungssystemen ein Mehrfachflop verliehen werden, der Farbeffekt wird verstärkt und neuartige Farbeffekte werden erzielt.

Gegenstand der Erfindung ist somit ein Pigmentgemisch bestehend aus mindestens zwei Komponenten, wobei Komponente A mit ein oder mehreren Metalloxiden und/oder Metallen beschichtete SiO₂-Flakes und Komponente B plättchenförmige, nadelförmige oder sphärische Farbmittel oder Füllstoffe sind.

Gegenstand der Erfindung sind ebenfalls die Formulierungen, wie z. B. Farben, Lacke, Druckfarben, Kunststoffe, Pulverlacke und kosmetische Zubereitungen, die das erfindungsgemäße Pigmentgemisch enthalten.

Die beschichteten SiO₂-Flakes können in jedem Verhältnis mit dem Farbmittel gemischt werden. Vorzugsweise ist das Verhältnis von Komponente A zu Komponente B 1 10 bis 10 : 1, insbesondere 1 : 2 bis 2 : 1.

Die nach der WO 93/08237 auf einem endlosen Band hergestellten SiO₂-Flakes basieren auf einer plättchenförmigen, transparenten, farbigen oder farblosen Matrix und besitzen in der Regel eine Dicke zwischen 0,1 und 5 µm, insbesondere zwischen 0,2 und 2,0 µm. Die Ausdehnung in den beiden anderen Dimensionen beträgt üblicherweise zwischen 1 und 250 µm, vorzugsweise zwischen 2 und 100 µm, und insbesondere zwischen 5 und 40 µm. Die SiO₂-Flakes werden mit ein oder mehreren Metalloxidschichten und/oder Metallschichten versehen. Geeignete Metalloxide oder Metalloxidgemische sind beispielsweise Titandioxid, Zirkonoxid, Zinkoxid, Eisenoxide und/oder Chromoxid, insbesondere TiO₂ und/oder Fe₂O₃. Die Beschichtung der SiO₂-Flakes kann z.B. erfolgen wie in der WO 93/08237 (naßchemische Beschichtung) oder DE-OS-196 14 637 (CVD-Verfahren) beschrieben.

Anstelle der äußeren Metalloxidschicht kann auch eine semitransparente Schicht eines Metalls verwendet werden. Geeignete Metalle dafür sind beispielsweise Cr, Ti, Mo, W, Al, Cu, Ag, Au, oder Ni. Bevorzugte Pigmente weisen folgenden Schichtaufbau auf: SiO₂-Flakes + Metall + SiO₂ + Metalloxid.

Zur Erziehung spezieller Farbeffekte können in die hoch- bzw. niedrigbrechenden Metalloxidschichten zusätzlich noch feinteilige Partikel im Nanometergrößenbereich eingebracht werden. Als geeignet dafür erweisen sich beispielsweise feinteiliges TiO₂ oder feinteiliger Kohlenstoff (Ruß) mit Teilchengrößen im Bereich von 10 - 250 nm. Durch die lichtstreuenden Eigenschaften derartiger Partikel kann gezielt auf Glanz und Deckvermögen Einfluß genommen werden. Vorzugsweise sind die SiO₂-Flakes mit ein oder mehreren Metalloxiden beschichtet.

Als Komponente B für die erfindungsgemäße Pigmentmischung sind alle dem Fachmann bekannten nadelförmigen und sphärischen Farbmittel geeignet, die eine Partikelgröße von 0,001 bis 10 µm, vorzugsweise 0,01 bis 1 µm, aufweisen. Bevorzugt enthalten die erfindungsgemäßen Pigmentgemische als Farbmittel Absorptionsmittel und Füllstoffe.

Zu den sphärischen Farbmitteln zählen insbesondere TiO₂, eingefärbtes SiO₂, CaSO₄, Eisenoxide, Chromoxide, Ruß, organische Farbpigmente, wie z.B. Anthraquinon-Pigmente, Chinacridon-Pigmente, Diketopyrrolopyrrol-Pigmente, Phthalocyanin-Pigmente, Azopigmente, Isoindolin-Pigmente. Bei den nadelförmigen Pigmenten handelt es sich vorzugsweise um BiOCl, eingefärbte Glasfasern, α-Fe₃O₄, organische Farbpigmente, wie z.B. Azopigmente, β-Phthalocyanin Cl Blue 15,3, Cromophtalgelb 8GN (Ciba-Geigy), Irgalith Blau PD56 (Ciba-Geigy), Azomethinkupferkomplex CI Yellow 129, Irgazingelb 5GT (Ciba-Geigy).

Vorzugsweise enthalten kosmetische Formulierungen (insbesondere dekorative Formulierungen) neben Farbmitteln und SiO₂-Flakes als weiteren wichtigen Bestandteil Füllstoffe (einen oder mehrere) oder deren Gemische von Mengen von 1-50 %, insbesondere 1-30 %, ganz besonders bevorzugt 1-15 %.

Geeignet sind Füllstoffe wie Glimmer, Talkum, TiO₂ und Glimmer, TiO₂ + BaSO₄ + Glimmer, Glimmer + Silica, Silica, Silica + TiO₂ + Fe₂O₃, BiOCI, BiOCI + Mica, BiOCI + Talkum. Diese Produkte sind erhältlich unter den Markennamen der Fa. Merck KGaA: Ronasphere®, Ronasphere® LDP, Micronasphere® M, Silk Mica, Satin Mica, Naturaleaf Powder, Low Luster Pigment, Extender W, Talkum feinst gepulvert, Biron®, Bital® und Mibiron®, sowie Nylon-12 (Fa. Elf Atochem), Nylon-6 (Fa. Elf Atochem), Boron Nitride, Nylon Powder (Fa. Elf Atochem), Polyamide 12 (Fa. Kobo), Polyethylene (Fa. Kobo), PTFE (Fa. Presperse), Lauroyl Lysine (Fa. Ikeda), Polymethylmethacrylat (Fa. Ikeda), Calcium Aluminium Borosilicate (Fa. Presperse).

Die erfindungsgemäße Pigmentmischung ist einfach und leicht handzuhaben. Die Pigmentmischung kann durch einfaches Einrühren in das Anwendungssystem eingearbeitet werden. Ein aufwendiges Mahlen und Dispergieren der Pigmente ist nicht erforderlich.

Die erfindungsgemäße Pigmentmischung kann zur Pigmentierung von Lacken, Druckfarben, Kunststoffen, Agrarfolien, Saatgutbeschichtung, Lebensmitteleinfärbungen, Knopfpasten, Arzneimittelüberzügen oder kosmetischen Formulierungen verwendet werden. Die Konzentration der Pigmentmischung im zu pigmentierenden Anwendungssystem liegt in der Regel zwischen 0,1 und 70 Gew. %, vorzugsweise zwischen 0,1 und 50 Gew. % und insbesondere zwischen 1,0 und 10 Gew. %, bezogen auf den Gesamtfestkörpergehalt des Systems. Sie ist in der Regel abhängig vom konkreten Anwendungsfall.

Kunststoffe enthaltend das erfindungsgemäße Pigmentgemisch in Mengen von 0,01 bis 50 Gew. %, insbesondere 0,1 bis 7 Gew. %, zeichnen sich häufig durch einen besonderen Sparkle-Effekt aus.

Im Lackbereich, insbesondere im Automobillack, wird das Pigmentgemisch, auch für 3-Schichtaufbauten in Mengen von 0,1-10 Gew. %, vorzugsweise 1 bis 3 Gew. %, eingesetzt. Das Mischungsverhältnis der beschichteten SiO₂-Flakes mit Komponente B hängt vom gewünschten Effekt ab. Vorzugsweise werden die SiO₂-Flakes mit Komponente B im Verhältnis von 1 : 4, insbesondere von 1 : 3, eingesetzt.

Im Lack hat die erfindungsgemäße Pigmentmischung den Vorteil, daß der angestrebte Farbflop-Effekt durch eine einschichtige Lackierung (Einschichtsystem bzw. Base coat im 2-Schichtaufbau) erzielt wird. Dieser Farbflop ist auch bei diffusem Licht sehr deutlich ausgeprägt. Im Vergleich mit Lackierungen, die ein Interferenzpigment auf Basis von Glimmer enthalten statt der beschichteten SiO₂-Flakes, zeigen Lackierungen mit der erfindungsgemäßen Pigmentmischung eine deutlichere Tiefenwirkung und einen Glitzereffekt.

Das erfindungsgemäße Pigmentgemisch kann auch in der dekorativen und pflegenden Kosmetik eingesetzt werden. Die Einsatzkonzentration und das Mischungsverhältnis von SiO₂-Flakes mit Komponente B, insbesondere organischen und anorganischen Farbpigmenten und Farbstoffen, natürlichen oder synthetischen Ursprungs, wie z.B. Chromoxid, Ultramarin, sphärischen SiO₂- oder TiO₂-Pigmenten, sind abhängig vom Anwendungsmedium und dem Effekt, der erzielt werden soll. Die Mischung aus SiO₂-Flakes mit anderen Pigmenten kann in allen Verhältnissen erfolgen, vorzugsweise beträgt das Verhältnis 1 : 10 bis 10 : 1. Die Einsatzkonzentration reicht von 0,01 Gew. % im Shampoo bis zu 70 Gew. % im Preßpuder. Bei einer Mischung von beschichteten SiO₂-Flakes mit sphärischen Füllstoffen, z. B. SiO₂, kann die Konzentration bei 0,01-70 Gew. % in der Formulierung liegen. Die kosmetischen Produkte, wie z.B. Nagellacke, Lippenstifte, Preßpuder, Shampoos, lose Puder und Gele, zeichnen sich durch besonders interessante Glanz- und/oder Farbeffekte aus. Der Glitzereffekt in Nagellack kann gegenüber herkömmlichen Nagellacken mit Hilfe der erfindungsgemäßen Pigmentgemische deutlich gesteigert werden. Weiterhin kann das erfindungsgemäße Pigmentgemisch in Badezusätzen, Zahnpasten und zur Veredlung von Lebensmitteln, z. B. Masse-Einfärbung oder als Überzug, eingesetzt werden.

Bei der Pigmentierung von Bindemittelsystemen z. B. für Farben und Druckfarben für den Tiefdruck, Offsetdruck oder Siebdruck, oder als Vorprodukt für Druckfarben, z.B. in Form von hochpigmentierten Pasten, Granulaten, Pellets, etc., haben sich insbesondere Pigmentgemische bestehend aus beschichteten SiO₂-Flakes mit sphärischen Farbmitteln, wie z.B. TiO₂, Ruß, Chromoxid, Eisenoxid sowie organische Farbpigmente, als besonders geeignet erwiesen. Das Pigmentgemisch wird in der Regel in die Druckfarbe in Mengen von 2-35 Gew. %, vorzugsweise 5-25 Gew. %, und insbesondere 8-20 Gew. % eingearbeitet. Offsetdruckfarben können das Pigmentgemisch bis zu 40 Gew. % und mehr enthalten. Die Vorprodukte für die Druckfarben, z.B. in Granulatform, als Pellets, Briketts, etc., enthalten neben dem Bindemittel und Additiven bis zu 95 Gew. % des erfindungsgemäßen Pigmentgemisches. Das Mischungsverhältnis von Komponente A zu Komponente B liegt vorzugsweise im Bereich von 1 : 10 bis 10 : 1. Die Druckfarben enthaltend das erfindungsgemäße Pigmentgemisch zeigen reinere Farbtöne und sind aufgrund der guten Werte für die Viskosität besser verdruckbar.

Gegenstand der Erfindung sind somit auch Formulierungen enthaltend das erfindungsgemäße Pigmentgemisch.

Die nachfolgenden Beispiele sollen die Erfindung erläutern, ohne sie jedoch zu begrenzen.

### Beispiele

### Beispiel 1: - Druckfarbe

Das Pigment wurde bei 600 Upm in das lösemittelhaltige Bindemittel eingerührt und die Druckfarben wurden anschließend auf Schwarz-Weiß-Karten aufgerakelt.

### Farbe Nr. 1:

88,0 g Gebr. Schmidt 95 MB 011 TW
10,0 g mit Fe₂O₃ beschichtete SiO₂-Flakes der Teilchengröße 5 bis 40 µm
2,0 g Gebr. Schmidt 95 MB 022-TW (Grün)

### Farbe Nr. 2: Vergleich

88,0 g Gebr. Schmidt 95 MB 011 TW
10,0 g mit Fe₂O₃ beschichteter Glimmer der Teilchengröße 10 bis 60 µm
2,0 g Gebr. Schmidt 95 MB 022-TW (Grün)

Die Farbkarte mit der Farbe Nr. 1 zeigt visuell einen deutlich besseren Farbflop als die Farbkarten mit der Vergleichsfarbe Nr. 2.

### Beispiel 2: - Autolack

| | |
|---|---|
| 2,0 g | mit Fe₂O₃ beschichtete SiO₂-Flakes der Teilchengröße 5-40 µm |
| 1,5 g | Heliogenblau L 6930 |
| 0,2 g | Hostapermgrün 8G |
| 0,05 g | Farbruß FW 200 |
| 66,6 g | Basislack (A4) MP-System (FK = 19 %) |
| 29,65 g | Verdünnermischung |

Es wird ein Farbton mit einem stark ausgeprägten Blau-Grün-Umschlag und goldenen Highlights erreicht, der sogar bis Richtung Dunkelviolett changieren kann.

### Beispiel 3: - Kunststoff

Den Kunststoffgranulaten Polypropylen PP Stamylan PPH10 (Fa. DSM) bzw. Polystyrol 143E (Fa. BASF) werden jeweils
a) 1 % mit Fe₂O₃ beschichtete SiO₂-Flakes der Teilchengröße 5 - 40 µm
b) eine Mischung aus
   1 % mit Fe₂O₃ beschichtete SiO₂-Flakes der Teilchengröße 5 - 40 µm und 0,1 % PV-Echtblau B2G01 (Pigment Blue 15,3 der Fa. Clariant)
c) 1 % mit Fe₂O₃ beschichteter Glimmer der Teilchengröße 10 - 60 µm
d) eine Mischung aus
   1 % mit Fe₂O₃ beschichteter Glimmer der Teilchengröße 10- 60 µm und 0,1 % PV-Echtblau B2G01 (Pigment Blue 15,3 der Fa. Clariant)
zugegeben.

Das pigmentierte Granulat wird anschließend auf einer Spritzmaschine zu Stufenplättchen verarbeitet. Die Unterschiede zwischen den beiden Kunststoffen sind gering, so daß in der nachfolgenden Tabelle nur eine für beide Materialien gültige qualitative Aussage gemacht wird:

| | Versuch 3a | Versuch 3b | Versuch 3c | Versuch 3d |
|---|---|---|---|---|
| Farbe im Glanzwinkel nahe 0° | rötlich | rötlich-grau | gelblich | grünlich |
| Farbe im Glanzwinkel nahe 80° | gelblich bräunlich | gelbgrün | rötlich | blaugrün |

Die Kombination eines Blaupigments mit jeweils einem Glanzpigment (SiO₂ oder Glimmerbasis) verdeutlicht recht gut wie unterschiedlich das Kippverhalten der beiden Glanzpigmente ist.

### Beispiel 4: - Lidschatten

| Phase A | |
|---|---|
| 5,00 % | Ronasphere® (Silica der Fa. Merck KGaA) |
| 25,00 % | mit TiO₂ beschichtete SiO₂-Flakes der Teilchengröße 5-40 µm (Fa. Merck KGaA) |
| 5,00 % | C.I. Pigment Green 18 (C177289) |
| 47,42 % | Talc |
| 7,18 % | Solanum Tuberosum (Potato Starch) |
| 2,40 % | Magnesium Stearate |

| Phase B | |
|---|---|
| 6,96 % | Isopropyl Stearate |
| 0,40 % | Cetyl Palmitate |
| 0,40 % | Petrolatum |
| 0,08 % | Konservierung |

Die Bestandteile der Phase A werden zusammengegeben und vorgemischt. Anschließend wird die Pudermischung unter Rühren tropfenweise mit der geschmolzenen Phase B versetzt. Die Puder werden bei 40-50 bar gepreßt.

### Beispiel 5: - Lippenstift

| Phase A | |
|---|---|
| 8,25 % | Cera alba |
| 4,95 % | Ceresin, Copernica Cerifera |
| 3,30% | Lanolin Oil |
| 5,28 % | Isopropyl Myristate |
| 1,98 % | Mineral Oil |
| 0,03 % | Tocopherol, Ascorbyl Palmitate, Ascorbic Acid, Citric Acid, PEG-8 |
| 0,06 % | Konservierung |
| 0,50 % | Aroma |
| 1,00 % | Lithalrubin BK, C.I. Pigment Red 57:1 (Cl 15850) |
| ad 100,00 % Ricinus Communis (20 % in Castor Oil) | |

| Phase B | |
|---|---|
| 2,00 % | Ronasphere® (Silica der Fa. Merck KGaA) |
| 15,00 % | mit Eixenoxid beschichtete SiO₂-Flakes der Teilchengröße 5-40 µm (Fa. Merck KGaA) |

Die Bestandteile der Phase A werden auf 75 °C erhitzt und aufgeschmolzen. Die Pigmente der Phase B werden zugegeben und alles gut durchgerührt. Die Lippenstiftmasse wird dann in der auf 65 °C temperierten Gießapparatur 15 Min. gerührt. Die homogene Schmelze wird in die auf 65 °C vorgewärmten Gießformen gegossen. Anschließend kühlt man die Formen ab und entfernt die Gießlinge kalt.

## Patentansprüche

1. Pigmentgemisch bestehend aus mindestens zwei Komponenten, wobei Komponente A mit ein oder mehreren Metalloxiden und/oder Metallen beschichtete SiO₂-Flakes und Komponente B plättchenförmige, nadelförmige oder sphärische Farbmittel oder Füllstoffe sind.

2. Pigmentgemisch nach Anspruch 1, **dadurch gekennzeichnet, daß** Komponente A mit TiO₂ und/oder Fe₂O₃ beschichtete SiO₂-Flakes sind.

3. Pigmentgemisch nach Anspruch 1 oder 2, **dadurch gekennzeichnet daß** Komponente B eingefärbte Glaspartikel, Ruß, Silica, organische Farbpigmente und/oder anorganische Farbpigmente sind.

4. Pigmentgemisch nach einem der Ansprüche 1 bis 3, **dadurch gekennzeichnet, daß** Komponente A und Komponente B im Verhältnis 10:1 bis 1:10 gemischt sind.

5. Verwendung des Pigmentgemisches nach Anspruch 1 in Farben, Lacken, insbesondere Automobillacken, Druckfarben, Kunststoffen, Pulverlacken, zur Saatguteinfärbung, in kosmetischen Formulierungen und zur Veredelung von Lebensmitteln.

6. Formulierungen enthaltend ein Pigmentgemisch nach Anspruch 1.

## Claims

1. Pigment mixture consisting of at least two components, where component A comprises SiO₂ flakes coated with one or more metal oxides and/or metals, and component B comprises platelet-shaped, needle-shaped or spherical colorants or fillers.

2. Pigment mixture according to Claim 1, **characterised in that** component A comprises SiO₂ flakes coated with TiO₂ and/or Fe₂O₃.

3. Pigment mixture according to Claim 1 or 2, **characterised in that** component B comprises coloured glass particles, carbon black, silica, organic coloured pigments and/or inorganic coloured pigments.

4. Pigment mixture according to one of Claims 1 to 3, **characterised in that** component A and component B are mixed in a ratio of from 10:1 to 1:10.

5. Use of the pigment mixture according to Claim 1 in surface coatings, paints, in particular automobile paints, printing inks, plastics, powder coatings, for colouring seed, in cosmetic formulations and for treating foods.

6. Formulations comprising a pigment mixture according to Claim 1.

## Revendications

1. Mélange de pigments constitué d'au moins deux composants, le composant A étant des lamelles de SiO₂ revêtues d'un ou plusieurs oxydes métalliques et/ou de métaux et le composant B étant des colorants ou des matières de charge sous forme lamellaire, en forme d'aiguille ou sphériques.

2. Mélange de pigments selon la revendication 1, **caractérisé en ce que** le composant A est constitué de lamelles de SiO₂ revêtues de TiO₂ et/ou de Fe₂O₃.

3. Mélange de pigments selon la revendication 1 ou 2, **caractérisé en ce que** le composant B est constitué de particules de verre colorées, de noir de fumée, de silice, de pigments organiques colorés et/ou de pigments inorganiques colorés.

4. Mélange de pigments selon l'une des revendications 1 à 3, **caractérisé en ce que** le composant A et le composant B sont dans un rapport compris entre 10:1 et 1:10.

5. Utilisation des mélanges de pigments selon la revendication 1 dans les couleurs, vernis, en particulier peintures pour carrosseries, encres d'imprimerie, matières plastiques, vernis en poudre, pour la coloration des semences, dans les formulations cosmétiques et pour la valorisation de denrées alimentaires.

6. Formulations contenant un mélange de pigments selon la revendication 1.
